(19) Europäisches Patentamt
European Patent Office
Office européen des brevets

(11) **EP 3 339 848 B1**

(12) **EUROPEAN PATENT SPECIFICATION**

(45) Date of publication and mention of the grant of the patent:
**02.12.2020 Bulletin 2020/49**

(51) Int Cl.:
*G01N 33/205* [(2019.01)]     *G01N 25/04* [(2006.01)]

(21) Application number: **16382651.4**

(22) Date of filing: **23.12.2016**

(54) **METHOD TO DETERMINE THE CARBON EQUIVALENT CONTENT OF A CAST IRON ALLOY HAVING A HYPEREUTECTIC COMPOSITION AND EQUIPMENT TO CARRY IT OUT**

VERFAHREN ZUR BESTIMMUNG DES KOHLENSTOFFÄQUIVALENTGEHALTS EINER GUSSEISENLEGIERUNG MIT EINER ÜBEREUTEKTISCHEN ZUSAMMENSETZUNG UND AUSRÜSTUNG ZUR DURCHFÜHRUNG DAVON

PROCÉDÉ DE DÉTERMINATION DU CONTENU ÉQUIVALENT CARBONE D'UN ALLIAGE DE FONTE AYANT UNE COMPOSITION HYPEREUTECTIQUE ET ÉQUIPEMENT POUR SA MISE EN ŒUVRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**27.06.2018 Bulletin 2018/26**

(73) Proprietor: **Fundación Azterlan**
**48200 Durango (Bizkaia) (ES)**

(72) Inventors:
• **DE LA TORRE, Urko**
**48200 Durango, Vizcaya (ES)**
• **BRAVO, Beňat**
**48200 Durango, Vizcaya (ES)**
• **MÉNDEZ, Susana**
**48200 Durango, Vizcaya (ES)**
• **LOIZAGA, Aitor**
**48200 Durango, Vizcaya (ES)**

(74) Representative: **Balder IP Law, S.L.**
**Paseo de la Castellana 93**
**5ª planta**
**28046 Madrid (ES)**

(56) References cited:
**US-A- 3 546 921     US-A- 4 008 604**
**US-A- 5 503 475     US-A- 5 577 545**

• **CHEN IG- ET AL: "COMPUTER-AIDED DIFFERENTIAL THERMAL ANALYSIS OF SPHEROIDAL AND COMPACTED GRAPHITE CAST IRONS", AFS TRANSACTIONS, AMERICAN FOUNDRY SOCIETY, US, vol. 92, April 1984 (1984-04), pages 947-964, XP001027200,**

**Description**

**TECHNICAL FIELD**

The present invention belongs to the cast iron alloys sector, and more particularly refers to methods for controlling the carbon content or carbon equivalent content for all composition ranges (hypoeutectic, eutectic and hypereutectic) by means of thermal analysis techniques, in order to ensure that the parts are produced with the desired carbon content. The invention also relates to a software product for carrying out the method and to an equipment for carrying it out.

**BACKGROUND OF THE INVENTION**

The manufacturing process of cast iron parts, considering lamellar, vermicular and ductile iron is being constantly improved in order to obtain castings with better mechanical properties, higher production yield, while minimizing the costs. Additionally, a weight reduction is sought above all on the automotive, railway and wind energy sectors.

All these premises set the basis for a process where the working limits must be every day narrower and with an exhaustive control.

Two of the most important parameters regarding the production of cast iron parts are the carbon and silicon contents, or said otherwise, the carbon equivalent *(%Ceq=%C+%Si/3)*. By controlling properly the value of this parameter, the feeding capacity of the alloy can be increased. This means that the feeding system of the pattern design can be minimized and this way the pattern yield and the production yield are increased. This leads to castings with less scrap rate and minimized costs.

To achieve the above mentioned goals, the current tendency regarding small castings production is to increase the carbon equivalent obtaining hypereutectic alloys. The carbon equivalent is increased by increasing the carbon content, because the expansion during the graphite precipitation helps to counteract the metallic contraction. The graphite density is decreased when solidifying compared to the liquid carbon. On the other hand, the solid austenite's density is higher than the density of the liquid melt. This is why the higher the graphite precipitation the lower the contraction tendency of the alloy.

The problem of this chemical composition change is that the control methodology becomes more complex. The methods to control the carbon equivalent in foundries are three:

- Spark spectrometry: This is the most accurate technique to measure in real time the most part of the elements of the alloy. The measurement time is very fast and the methodology is easy to carry out. But for the carbon content it is not very accurate.
- Combustion technique: The carbon measurement by means of this technique is very accurate, but it is not possible to measure other elements than sulphur and carbon. Additionally, the measurement time is high and the equipment is not available on all foundries due to its high cost.
- Thermal analysis: This technique is used to measure the carbon equivalent. The carbon measurement is very accurate and the measurement time fast. Besides, it is a cheap methodology. Additionally, this equipment allows obtaining some information regarding the metallurgic quality of the alloy. It is a very economical and robust implanted technique, which makes it ideal for the intensive production of parts.

Raising the carbon equivalent presents the operating problem that for hypereutectic alloys, the thermal analysis is not valid anymore. Some changes must be applied in order to make this methodology valid for these alloys, which is the aim of the present invention.

The carbon equivalent control is very important because at high temperatures and in the conditions of a foundry, the carbon content in the alloy evolves, as it reacts with oxygen (among others) and disappears from the alloy mixture.

The result is that its presence evolves so that between the initial mix in the furnace and the final part, there may be so high differences that the part has no longer the desired carbon content.

This can be particularly serious when it comes to parts intended for the automotive sector, for example in braking systems, where control of parameters is essential for safety reasons.

Therefore, knowing with precision the carbon equivalent content in the final product is essential.

The above-mentioned thermal analysis for determining the carbon equivalent content is well known, which consists in the analysis of the cooling curves, which allows determining transition temperatures, such as liquidus temperature (TL) and eutectic minimum temperature (TE). However, as will be discussed below, thermal analysis is currently limited in the eutectic zone.

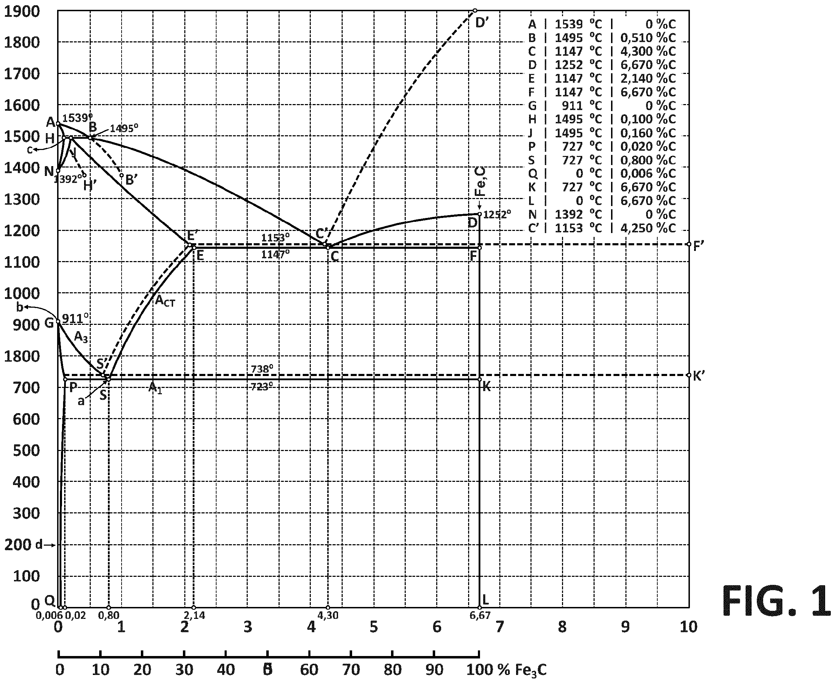

During the solidification process of a cast iron alloy, if the stable Fe-C diagram is followed, the phases austenite and graphite are formed (shown in Figure 1 with dashed lines). On the other hand, if iron carbides are formed instead of graphite, the solidification is performed following the metastable Fe-C diagram (shown in Figure 1 with straight lines).

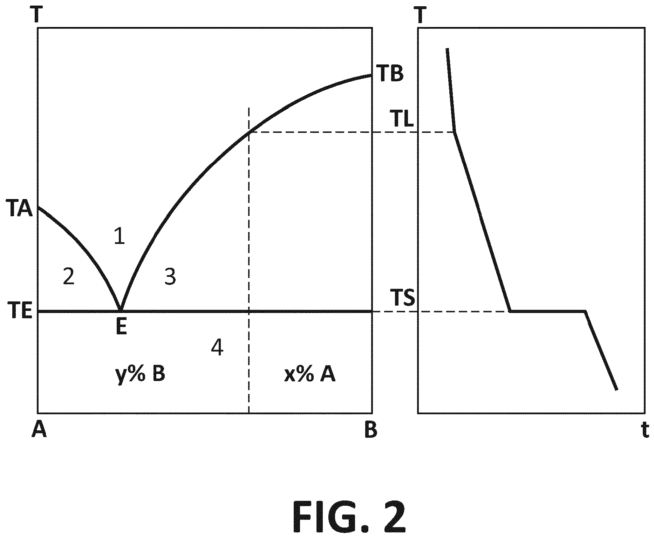

On the Figure 2 the relationship between the Fe-C diagram and a solidification curve obtained by means of thermal analysis are shown.

In order to calculate the carbon equivalent of a cast iron alloy by means of thermal analysis techniques, the conventional methodology is based on a metastable, also called white iron, solidification curve. The most important parameters of a white iron solidification curve, that is TL and TE, exhibit a linear relationship with the carbon and silicon contents.

However, this relationship is not valid for hypereutectic alloys. Considering that on the white solidification curve the eutectic point is displaced to higher carbon equivalent values, it can be considered that this conventional methodology is valid up to carbon equivalent values of around 4.60 %.

It is reminded that the carbon equivalent (considering only C and Si) is defined as:

$$\%Ceq = \%C + \%Si / 3 \qquad (I)$$

The conventional thermal analysis methodology to calculate the carbon equivalent for hypoeutectic and eutectic cast iron next described is based on the calculation system of the registered Thermolan® predictive system of the metallurgical quality. A similar description can also be found in the document "Thermal Analysis of Cast Iron" (Heraeus Electro-Nite), available at the site http://heraeus-electro-nite.com/:For (It is reminded, for the foregoing description, that a thermal analysis cup is poured with the liquid alloy only, whereas the composition cup contains tellurium for a lamellar iron alloy and tellurium plus a free magnesium neutralizer for ductile and vermicular iron alloys, which is mixed with the liquid alloy, thus obtaining white iron):

- So first a gray iron molten metal is poured in a composition cup and a white iron solidification curve is obtained, due to the carburizer effect of the Te. If working with ductile or vermicular iron alloys, a free magnesium neutralizer must also be added to the composition cup in order to enable the Te to apply its carburizer effect. The solidification curve is registered by means of a thermocouple placed on the thermal center of the cup.
- The liquidus temperature and the eutectic minimum temperature are defined from the solidification curve (See FIG. 2);
- The linear model that enables to obtain the carbon and silicon contents is the following (though new measures could improve their accuracy):

$$\%Si = 78.23 - 0.0683\ T_{eutetectic\ minimum} \qquad (II)$$

$$\%C = 0.0178\ T_{eutectic\ minimum} - 0.0084\ T_{liquidus} - 6.51 \qquad (III)$$

An aspect to take into account is that many variables intervene in the forms of the curves, so the models are different for each installation. However, there is certain uniformity, increasing as the technique progresses, which allows the results obtained in a particular installation to be used in different installations. This procedure allows not only performing many tests with the minimum cost, but also its results are very accurate, so that the repeatability of the tests is guaranteed.

But, as said before, this methodology is no longer valid for carbon equivalent values higher than 4.60 %. For values higher than that threshold, the linear model does not represent the reality and the higher the carbon equivalent, the higher the calculation error.

This deviation is due to the form of the Fe-C metastable diagram for the hypereutectic alloys, as shown in FIG. 1 (continuous lines), where the shape of the liquidus temperature curve changes abruptly.

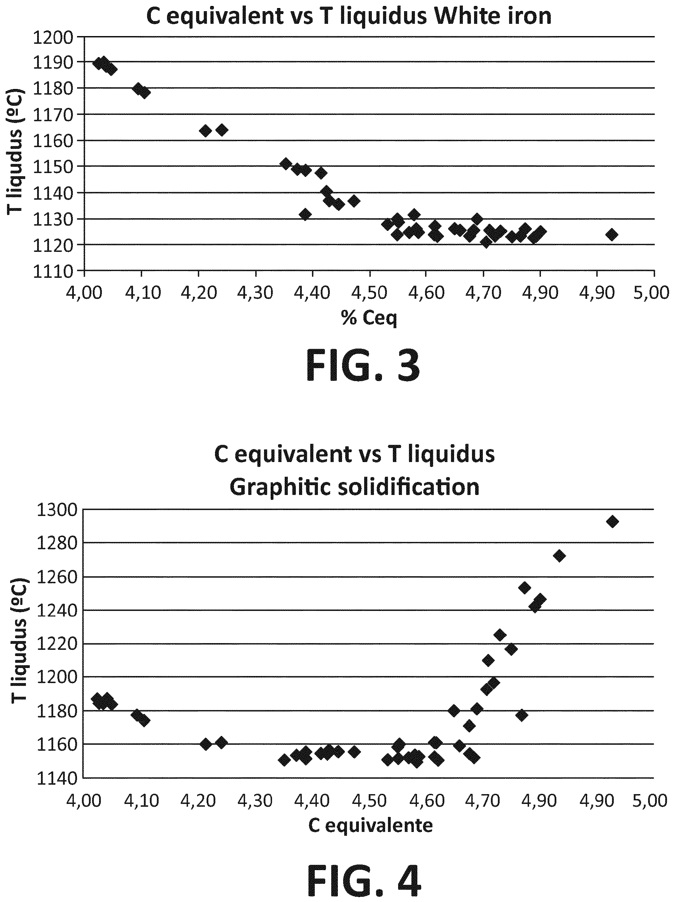

Experimentally, the next evolution of the white curve liquidus temperature with respect to the carbon equivalent was defined as shown in FIG. 3. The values of the carbon equivalent were defined by means of spark spectrometry and combustion techniques. In the hypoeutectic zone the higher the carbon content, the lower the liquidus temperature.

Here it is seen that for carbon equivalent values higher than 4.55 %, the value of the liquidus temperature remains more or less constant.

When reaching the eutectic composition the liquidus temperature becomes equal to the eutectic temperature, so that a shoulder can no longer be distinguished, and there is no longer a univocal relationship between liquidus temperature and carbon content, i.e. it is no longer possible to determine the carbon content. In other words, the model is no longer valid when, by increasing the carbon content and thus the carbon equivalent, the liquidus temperature descends until it gets confused with the eutectic temperature.

It is pointed out, however, that the relationship between the silicon content and the eutectic minimum temperature does not change.

Based on this fact, a necessity is seen of a new method to determine the carbon equivalent content higher than the threshold that defines the start of the eutectic metastable zone.

US3546921A discloses a method of production of an initial thermal arrest in the cooling curve of hypereutectic cast iron. US4008604A discloses a method for determination of carbon analysis in iron. US5577545A discloses a method for determination of the carbon equivalent in structure-modified cast iron. "COMPUTER-AIDED DIFFERENTIAL THERMAL ANALYSIS OF SPHEROIDAL AND COMPACTED GRAPHITE CAST IRONS" (Chen IG - et al.), AFS TRANSACTIONS, AMERICAN FOUNDRY SOCIETY, US, vol. 92, April 1984, pages 947-964 discloses a computer-aided differential thermal analysis of spheroidal and compacted graphite cast irons. US5503475A discloses a method for determination of the carbon equivalent, carbon content and silicon content of molten cast iron.

## DESCRIPTION OF THE INVENTION

For overcoming the state of the art limitations, the present invention proposes a method to determine the carbon equivalent content of a cast iron alloy which comprises the following steps:

**a)** previously determining the maximum carbon equivalent value for the validity of a linear thermal analysis model for hypoeutectic and eutectic zones that relates the carbon equivalent with the liquidus temperature and the eutectic minimum temperature of a white iron solidification curve. This step is equivalent to previously determining the concentration of carbon equivalent for which the liquidus shoulder disappears in the solidification curve of a white iron;

b) previously determining the minimum carbon equivalent value for the validity of a linear thermal analysis model for hypereutectic zone that relates the carbon equivalent with the liquidus temperature for a graphitic solidification curve and the eutectic minimum temperature of a white iron solidification curve; in other words, to previously determine in the hypereutectic zone the concentration of carbon equivalent (or the corresponding temperature) for which a liquidus shoulder appears on the graphitic solidification curve. Therefore, since the two models lose their validity at a certain distance of the eutectic point, the eutectic point corresponds in practice to an extended point, i.e. the skilled person must decide in which moments the transitions between the two models occur;

c) pour a liquid first sample of iron alloy in a composition cup and a liquid second sample of the iron alloy in a thermal cup; therefore a white iron sample and a graphitic iron sample are simultaneously obtained;

d) Obtain the solidification curve for both the first sample and the second sample and hence the white iron solidification curve and the graphitic iron solidification curve, respectively;

e) Obtain from the solidification curve of the first sample the liquidus temperature and the eutectic minimum temperature, and with these two values, define the carbon equivalent content for the iron alloy by means of the linear thermal analysis model for hypoeutectic and eutectic zones;

f) If the carbon equivalent content for the iron alloy obtained in step e) is lower than the maximum carbon equivalent value defined on the step a), then establish the carbon equivalent content of the iron alloy by using the linear thermal analysis model for hypoeutectic and eutectic zones;

g) If the carbon equivalent content for the iron alloy obtained in step e) is higher than the maximum carbon equivalent value defined in step a) then obtain the liquidus temperature of the second sample and check if it s higher than the value of the liquidus temperature corresponding to the minimum carbon equivalent value defined in step b);

h) If the result of step g) is positive, then establish the carbon equivalent content of the cast iron alloy by using the linear thermal analysis model for the hypereutectic zone;

i) If the result of step g) is negative, then establish the carbon equivalent as the conventional for the hypoeutectic and eutectic zones.

This method improves the accuracy of the current thermal analysis methods for determination of the carbon equivalent content in hypereutectic compositions.

In addition, the use of thermal analysis results, as highlighted in the background section, reliable and inexpensive, so that the technique can be carried out in facilities accustomed to using it, not having to become familiar with more complex and expensive equipment.

Furthermore, it is an easily automated method, which can be the object of a software that can be used in any installation, i.e. it is not left to chance by a particular technician. Therefore, repeatability is guaranteed.

In some embodiments, steps a) and b) are carried out by obtaining the solidification curves for multiple alloys having different carbon equivalent contents and determining the carbon and silicon contents by using techniques not making use of the solidification curves. These techniques are preferably spark spectrometry for determining the silicon content and combustion for determining the carbon content. However other suitable techniques can be used or already existing models can be used.

In some embodiments, step e) is carried out by determining the first inflexion point occurring before the eutectic plateau or, which is equivalent, by detecting a peak in the first derivative higher than a predetermined threshold value before the peak described for the eutectic plateau. Additionally, obtain the eutectic minimum temperature, by detecting the first null point or peak of the first derivative on the eutectic plateau.

The solidification curves are the time evolution of the temperature. These curves are obtained using the aforementioned thermal and composition cups, and sampling the temperature, for example using the equipment disclosed below. Obviously, it implies that points are obtained that are to be then fitted by, for example, with a polynomic model. Then the liquidus temperature corresponds to the point where the second derivative is null.

In some embodiments, step g) is carried out by determining the first inflexion point occurring before the eutectic plateau or by detecting a peak in the first derivative higher than a predetermined threshold value before the eutectic plateau. This technique is easily programmable.

In some embodiments, the linear thermal analysis model for hypoeutectic and eutectic zones that relates the carbon content with the liquidus temperature (TL) and the eutectic minimum temperature (TE) for a white iron is:

$$\%C = 0.0178 \cdot TE - 0.0084 \cdot TL - 6.51 \qquad (III)$$

In some embodiments, the linear thermal analysis model for hypoeutectic and eutectic -zones that relates the silicon content with the eutectic minimum temperature (TE) for a white iron is:

$$\%Si = 78.23 - 0.0683 \cdot TE \qquad (II)$$

It is pointed out that the silicon content is calculated in this way for the whole range of compositions.

In some embodiments, the linear model for hypereutectic zone that relates the carbon content with the liquidus temperature of a graphitic solidification curve (TLS) and the eutectic minimum temperature of a white iron solidification curve (TE):

$$\%C = 0.0082 \cdot TE - 0.0019 \cdot TLS - 7.382 \qquad (IV)$$

In some embodiments, the liquidus temperature of a graphitic solidification curve (TLS) corresponding to the minimum carbon equivalent value valid for the hypereutectic calculation methodology is 1164$\underline{o}$C.

It should be noted that the linear models for the hypo/eutectic and hypereutectic zones can be theoretically determined. However, many variables are involved in its determination, so the results in different installations will be different. It may also happen that other components, albeit in other quantities, are added to the alloy, whereby the models will also differ.

However, it is clear that if the measurements are carried out correctly, the models obtained will be very similar for different installations. In particular, the inventors have determined the two linear models (II and IV) for said hypo/eutectic and hypereutectic zones which give very good results when applied to other facilities.

The invention also relates to a method to determine the carbon equivalent content of a cast iron alloy having a hypereutectic composition which comprises the following steps:

a) pour a liquid first sample of the iron alloy in a composition cup and a liquid second sample in a thermal cup;
b) obtain the solidification curve for both the first sample and the second sample;
c) if the liquidus temperature can be determined for both samples (the first derivative has a maximum above a threshold value before the maximum related to the eutectic plateau) and they are not close (for example by establishing a practical threshold) to the eutectic minimum temperature then apply a linear thermal analysis model for hypoeutectic and eutectic zones that relates the carbon equivalent with the liquidus temperature and the eutectic minimum temperature of a white iron solidification curve and obtain the corresponding carbon equivalent content;
d) If the liquidus temperature can only be determined for the first sample (the first derivative has a maximum above a threshold value before the maximum related to the eutectic plateau) and is close (for example by establishing a practical threshold) to the eutectic minimum temperature and the second sample does not describe the liquidus temperature (the first derivative has only a maximum related to the eutectic minimum plateau), then apply a linear thermal analysis model for hypoeutectic and eutectic zones that relates the carbon equivalent with the liquidus temperature and the eutectic minimum temperature of a white iron solidification curve and obtain the corresponding carbon equivalent content.
e) If the liquidus temperature cannot be determined for neither of the samples (the first derivative has only a maximum related to the eutectic minimum plateau) then apply a linear thermal analysis model for hypoeutectic and eutectic zones.
f) If the liquidus temperature can only be determined for the second sample and is above a determined threshold value, then apply a linear thermal analysis model for hypereutectic zone.

The invention also relates to an equipment for determining the carbon equivalent content of a cast iron alloy having a hypereutectic composition, which comprises a composition cup, a thermal cup, both provided with temperature probes, a processing device for processing the measures taken with the probes, a base for holding the cups and for establishing a measuring circuit between the cups and the processor, the processing device being programmed for carrying out the calculation steps of the inventive method disclosed according to any of the variants described above.

**BRIEF DESCRIPTION OF THE DRAWINGS**

To complete the description and in order to provide for a better understanding of the invention, a set of drawings is provided. Said drawings form an integral part of the description and illustrate an embodiment of the invention, which should not be interpreted as restricting the scope of the invention, but just as an example of how the invention can be carried out. The drawings comprise the following figures:

FIG. 1 is a schematic representation of the main zones in the Fe-C diagram useful for understanding the present invention.
FIG. 2 shows the general shape of a cooling curve based on the Fe-C diagram that allows detecting the liquidus and the eutectic temperatures.
FIG. 3 is a liquidus temperature vs carbon equivalent content for a white iron alloy.
FIG. 4 is a liquidus temperature vs carbon equivalent content representation for a graphitic iron alloy.
FIG. 5 shows an equipment for carrying out the methods herein disclosed.
FIG. 6 is a flowchart of the method herein disclosed for determining the carbon content of a hypereutectic cast iron alloy.
FIG. 7 and 8 are respectively the cooling curves for the thermal sample and the composition sample in the hypoeutectic zone, with their first and second derivative also shown.
FIG. 9 and 10 are respectively the cooling curves for the thermal sample and the composition sample for a first composition in the eutectic zone with their first and second derivative also shown.
FIG. 11 and 12 are respectively the cooling curves for the thermal sample and the composition sample for a second composition in the eutectic zone with their first and second derivative also shown.
FIG. 13 and 14 are respectively the cooling curves for the thermal sample and the composition sample for a first composition in the hypereutectic zone with their first and second derivative also shown.
FIG. 15 and 16 are respectively the cooling curves for the thermal sample and the composition sample for a second composition in the hypereutectic zone with their first and second derivative also shown.

**DESCRIPTION OF A WAY OF CARRYING OUT THE INVENTION**

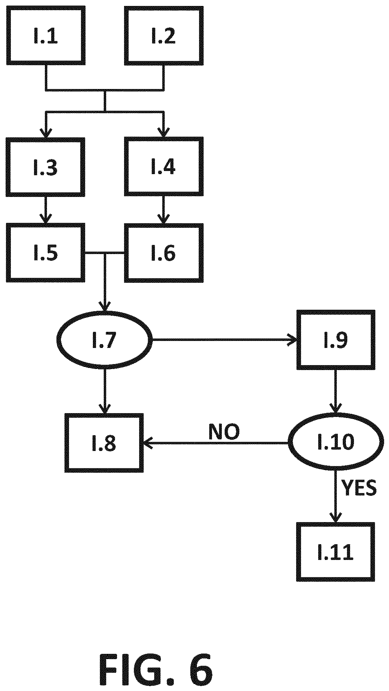

As shown in FIG. 6, the method herein disclosed is aimed at determining the carbon equivalent content of a cast iron alloy having a hypereutectic composition by means of thermal analysis techniques. Prior to analyzing the sample itself, the following two previous have been carried out, either in the same facility where the sample is analyzed or in another facility:

a) determining I.1 the maximum carbon equivalent value of validity of a linear model for hypoeutectic and eutectic zones that relates the carbon equivalent with the liquidus temperature and the eutectic minimum temperature for a white iron solidification curve.
b) determining I.2 the minimum value of the liquidus temperature of a graphitic solidification curve of validity of a linear model for hypereutectic zone that relates the carbon equivalent with the liquidus temperature for a graphitic solidification curve and the eutectic minimum temperature of a white solidification curve.

Then, for a particular sample, the following steps, which sequence is shown in FIG. 6, are carried out:

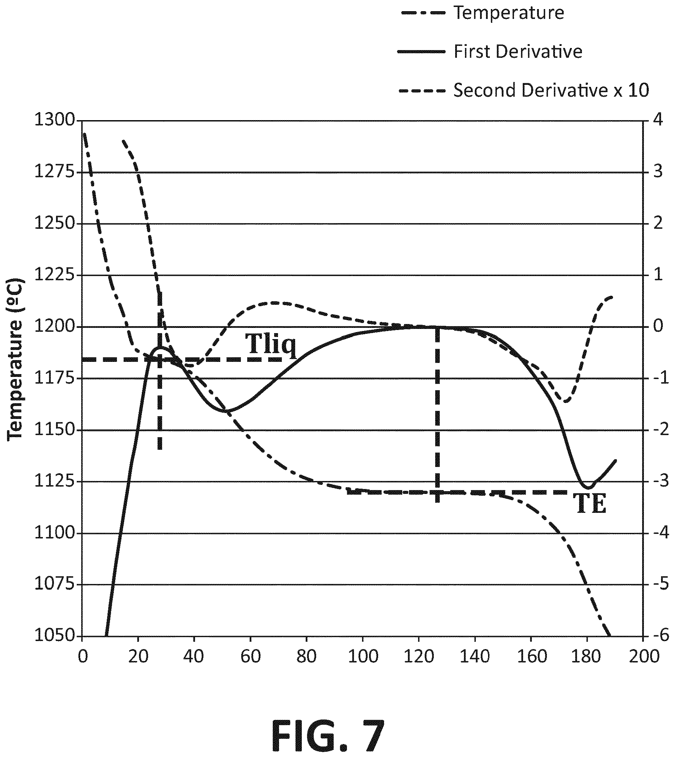

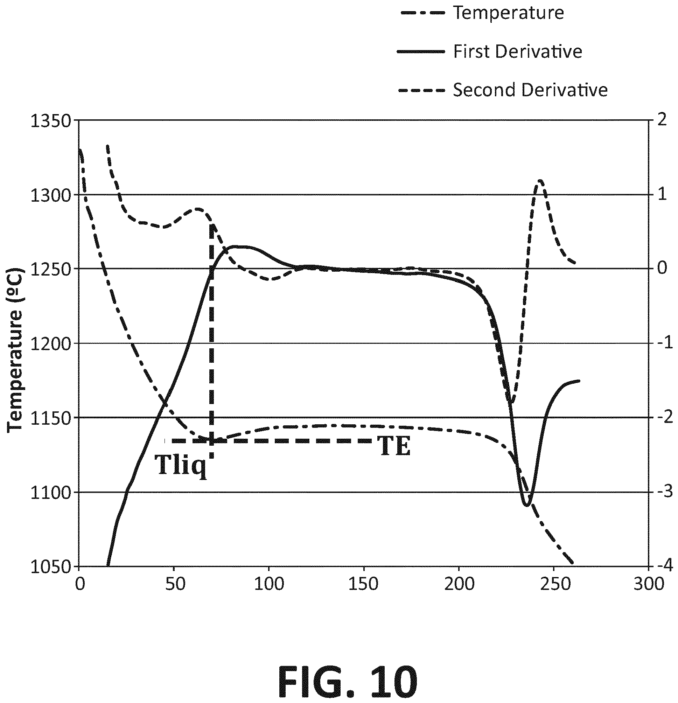

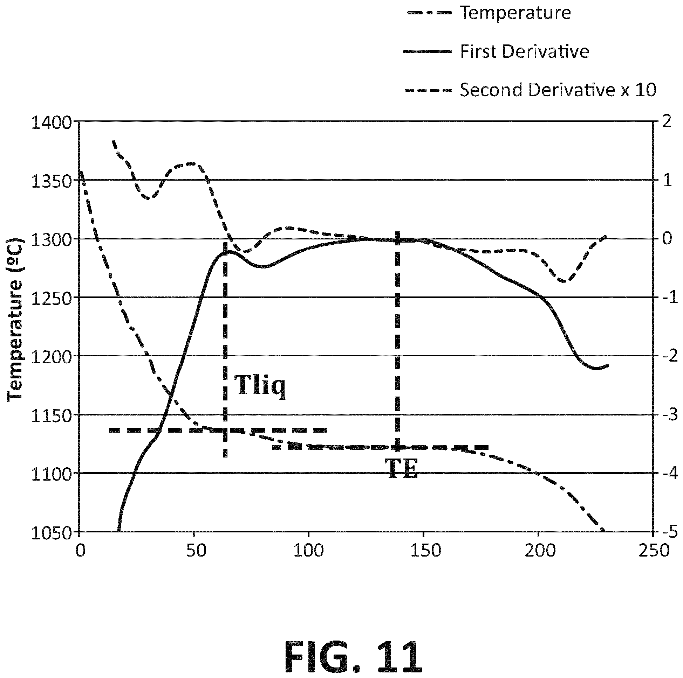

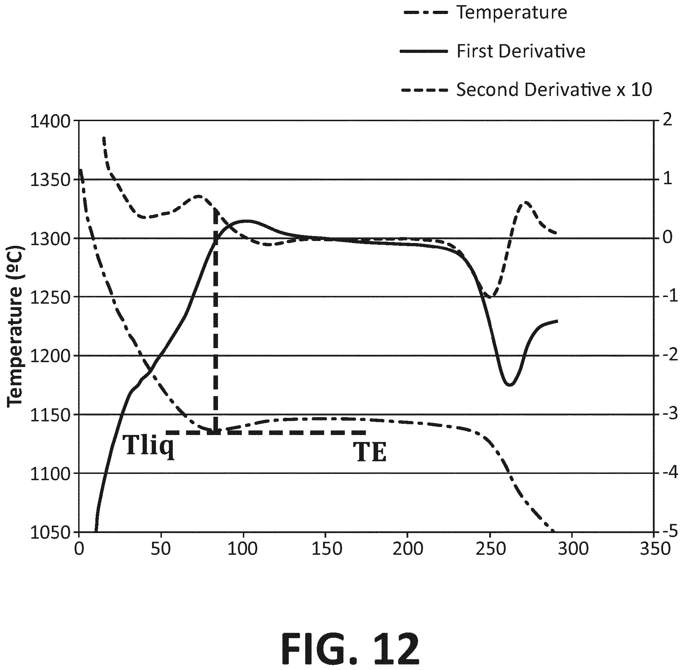

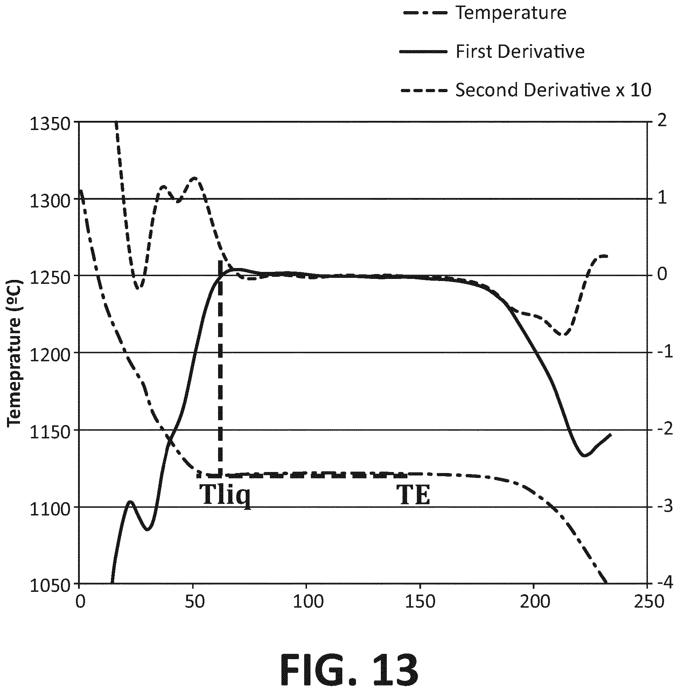

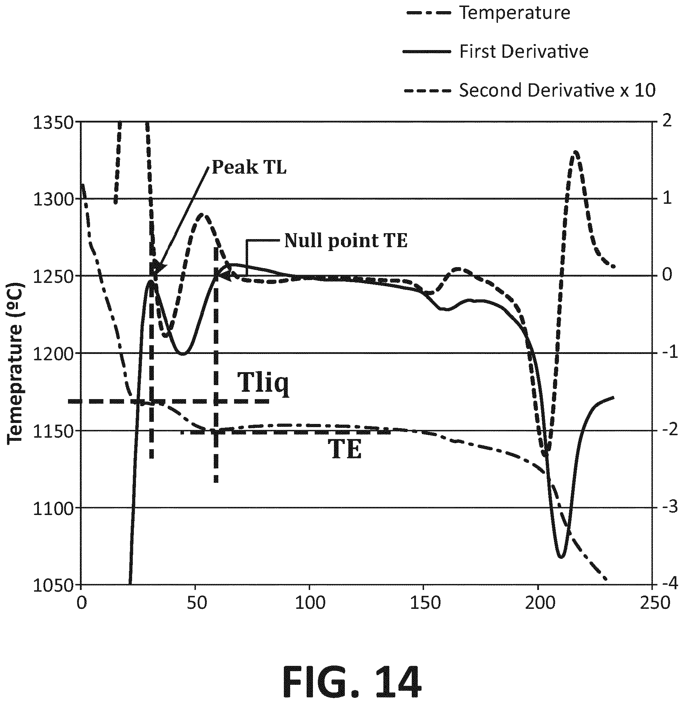

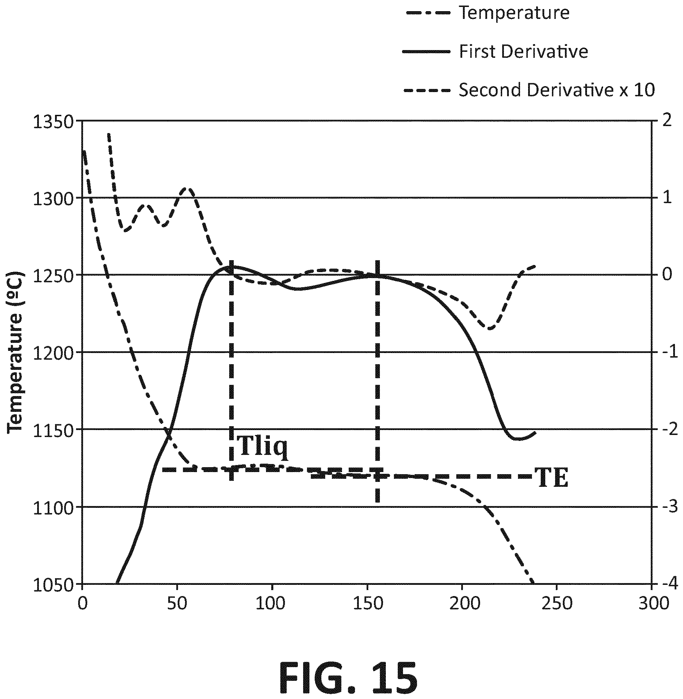

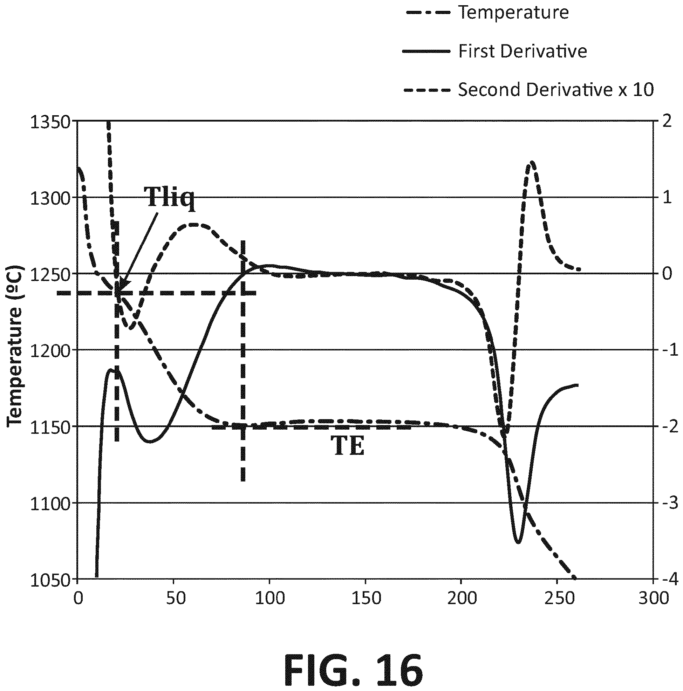

c) Pour a first sample of the liquid alloy in a composition cup I.3 and a second sample in a thermal cup I.4.
d) Obtain I.5, I.6 the solidification curve for both the first sample CC and the second sample TC; Figures 7 to 16 show real curves obtained for five different compositions, first a hypoeutectic sample, then two eutectic samples, with increasing equivalent carbon content, and then two hypereutectic samples, again with increasing carbon equivalent content.
e) Obtain from the solidification curve of the first sample the liquidus temperature. This temperature can be determined (FIGS. 7, 9, 11, 13, 15) by detecting the first appearance of an inflexion point occurring before the eutectic plateau of the solidification curve or by detecting a peak in the first derivative higher than a predetermined threshold value and before the peak described for the eutectic plateau. Additionally, obtain from the first sample the eutectic minimum temperature, by detecting the first null point or peak of the first derivative on the eutectic plateau. With this two values, define the carbon equivalent by means of a model for hypoeutectic and eutectic zones. A suitable model is given by: $\%C = 0.0178 \cdot TE - 0.0084 \cdot TL - 6.51$ (III) and $\%Si = 78.23 - 0.0683 \cdot TE$ (II). The first and second derivatives are shown in the time representations for each sample/cup.

f) If (comparison step I.7 of FIG. 6) the carbon equivalent content obtained in e) is lower than the maximum carbon equivalent value defined on step a) (arrow going downwards), then establish the carbon equivalent content of the cast iron alloy as the one obtained on step e) step I.8.

g) If the carbon equivalent content obtained in e) is higher (arrow to I.9) than the maximum carbon equivalent value, then obtain I.9 the liquidus temperature of the second sample and check I.10 if it is higher than the temperature defined on step b), which in the present practical case is established as 1164ºC. This liquidus temperature is calculated by determining the first inflexion point occurring before the eutectic plateau or by detecting a peak in the first derivative higher than a predetermined threshold value and before the peak described for the eutectic plateau.

h) If the result of step g) is positive, then (arrow going downwards) establish the carbon equivalent content of the cast iron alloy by using the linear model for hypereutectic zone I.11. The inventors have determined experimentally that a suitable model is given by: $\%C = 0.0082 \cdot TE - 0.0019 \cdot TLS - 7.382$ (IV) and $\%Si = 78.23 - 0.0683 \cdot TE$ (II).

i) If the result of step g) is negative (1.10 arrow going leftwards), then establish the carbon equivalent as the value obtained on step e) (1.8)

Now the ten cooling curves, corresponding to five different compositions, will be disclosed, which are aimed at showing the different results that can be obtained as a function of the carbon content:

In all the following cases, steps I.1 and I.2 have already been carried out, and the solidification curves have been determined I.3-I.6 of the cast iron alloy in question.

Case 1 - FIG.s 7 and 8 - HypoEutectic: %C = 3.47 ; %Si = 1.76 ; % **Ceq = 4.05%**

In this case, the composition is clearly in the hypoeutectic zone, and then both curves CC, TC allow to easily and unambiguously determining the Tliq.

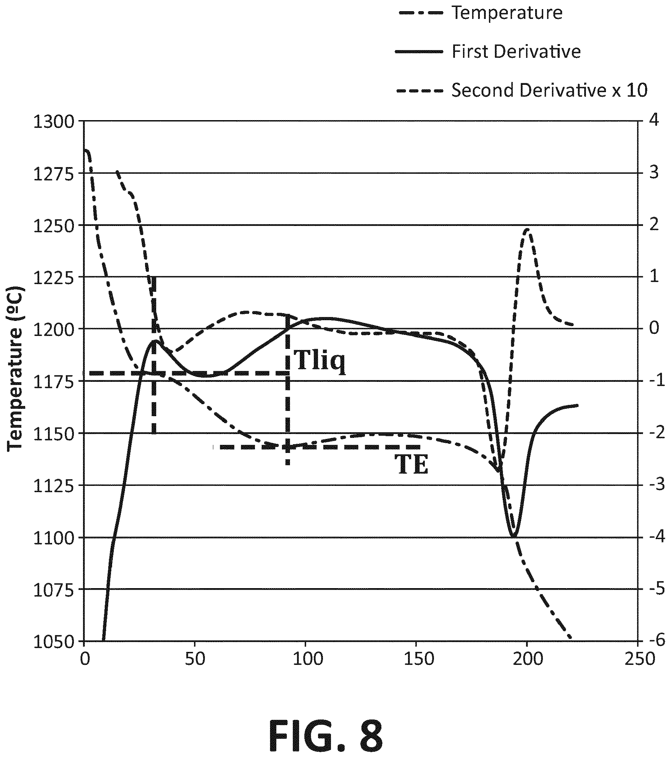

As shown in FIG. 7, the solidification of the composition cup (white iron) shows a liquidus shoulder (intersection of the auxiliary dashed lines, the vertical one showing the maximum of the first derivative, the horizontal one indicating the value of the liquidus temperature) at medium-high temperatures, which means that the alloy is in the hypoeutectic zone. On the other hand, as shown in FIG. 8, the thermal cup shows a liquidus temperature that is situated more or less at the height of the liquidus temperature of the composition cup. The calculation model for hypoeutectic and eutectic alloys is applied.

Case 2 - FIG.s 9 and 10 - Eutectic: %C = 3.99 ; %Si = 1.35 ; % **Ceq = 4.44%**

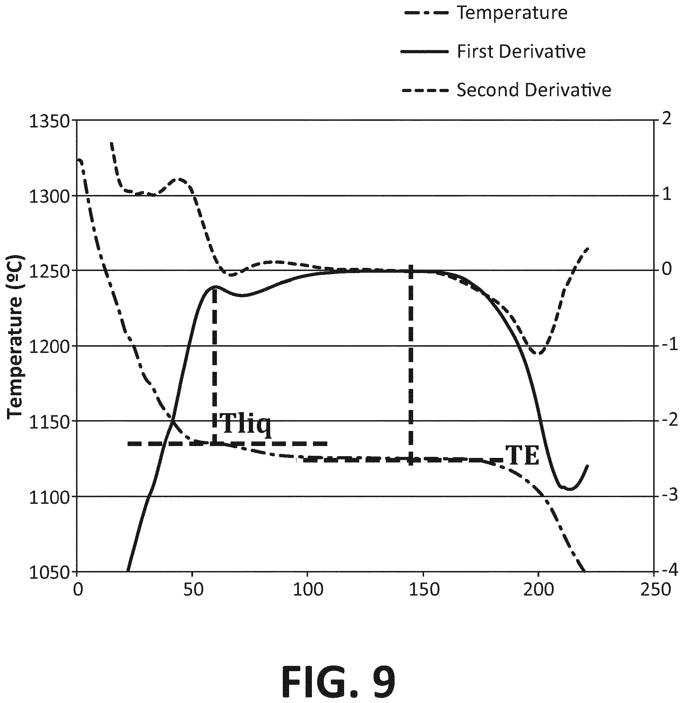

Figure 9 shows how the first maximum of the first derivative and the maximum corresponding to the eutectic plateau correspond to temperature values in the solidification curve (continuous blue line) very close to each other (For example <15ºC, although the skilled person can adjust it in function of improvements in the measurements accuracy), so that the cast alloy is considered as eutectic, and then, as explained above, the liquidus can be defined, although it is considered close to the eutectic point. In the thermal curve, however, both the liquid and minimum eutectic temperatures coincide with the same first null point of the first derivative. Then it is concluded that Ceq is eutectic.

The calculation model for hypoeutectic and eutectic alloys is applied.

Case 3 - FIG.s 11 and 12 - Eutectic: %C = 3.92 ; %Si = 1.60 ; % **Ceq = 4.45%**

Case 3 is analogous to case 2, but with different carbon and silicon contents that lead to an almost identic Ceq. The calculation model for hypoeutectic and eutectic alloys is applied.

Case 4 - FIG.s 13 and 14 - HyperEutectic: %C = 4.09 ; %Si = 1.74 ; % **Ceq = 4.67%**

In this case it is observed how the liquidus of the composition curve and the minimum eutectic correspond to the same first null point of the first derivative. The first maximum of the first derivative has a very low value and cannot be considered as the relative one to a high liquidus temperature. In other words, it can be seen that the composition curve does not show any "marked" liquidus shoulder, that is, the liquidus is at the level of the minimum eutectic temperature.

However, in the thermal cup it is observed that the first derivative exhibits two maxima, the one relative to the hypereutectic liquidus (Peak TL) and the minimum eutectic temperature plateau. These maxima correspond to the solidification curve (continuous blue line) with well-differentiated temperature values. And it is observed that the liquid is above 1164 ºC, which is a value that has been set by the inventors as the lower limit of this variable for the inventive method.

In other words, the results concerning the composition cup lead to the next step consisting in determining whether the cast alloy is at either the eutectic point or the hypereutectic zone. To determine this question the liquidus of the thermal curve is checked. Since it exhibits a "marked" liquidus shoulder, that is, since the first derivative has a peak before the peak corresponding to the eutectic plateau, it is considered to be in the hypereutectic zone. Therefore, the C% is calculated using a linear model in the hypereutectic zone.

Case 5 - FIG.s 15 and 16 - HyperEutectic: %C = 4.25 ; %Si = 1.46 ; % **Ceq = 4.75%**

Case 5 is analogous to case 4, but here it is appreciated that the shoulder shows up at a higher temperature.

The invention also refers to an equivalent method for a cast iron alloy having a hypereutectic composition comprising the steps of:

a) Pour a first sample of the liquid alloy in a composition cup and a second sample in a thermal cup;

b) Obtain the solidification curve for both the first sample and the second sample;

c) If the liquidus temperature can be determined for both samples (the first derivative has a maximum above a threshold value before the maximum related to the eutectic plateau) and they are not close (for example by establishing a practical threshold) to the eutectic minimum temperature then apply a linear conventional model for hypoeutectic and eutectic zones that relates the carbon equivalent with the liquidus temperature and the eutectic minimum temperature for a white iron solidification curve and obtain the corresponding equivalent carbon content;

d) If the liquidus temperature of the first sample can be determined (the first derivative has a maximum above a threshold value before the maximum related to the eutectic plateau) and it is close (for example by establishing a practical threshold) to the eutectic minimum temperature and the second sample does not describe the liquidus temperature (the first derivative has only a maximum related to the eutectic minimum plateau), then apply a linear conventional model for hypoeutectic and eutectic zones.

e) If the liquidus temperature cannot be determined for neither of the samples (the first derivative has only a maximum related to the eutectic minimum plateau) then apply a linear conventional model for hypoeutectic and eutectic zones. If the liquidus temperature can be determined for both samples (first sample's liquidus temperature must be very close to the eutectic point (for example by establishing a practical threshold), ensuring that the carbon equivalent is not hypoeutectic, and the second sample's liquidus is above a determined threshold value) then apply a linear model for hypereutectic zone that relates the carbon equivalent with the liquidus temperature of a graphitic solidification curve and the eutectic mimimum temperature of a white iron solidification curve.

f) If the liquidus temperature can only be determined for the second sample and it is above a determined threshold value, then apply a linear model for hypereutectic zone.

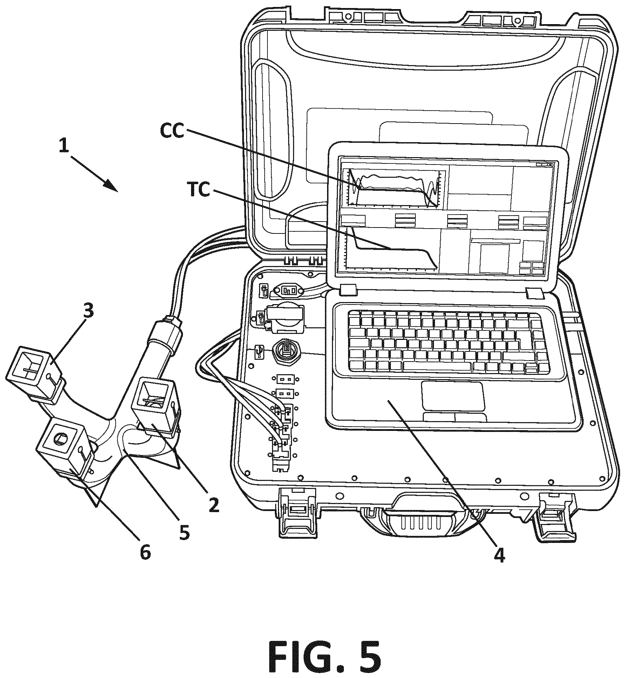

As shown in FIG. 5, the invention also relates to an equipment 1, preferably portable, for determining the carbon equivalent content of a cast iron alloy having a hypereutectic composition, which comprises a composition cup 2, a thermal cup 3, both provided with temperature probes, a processing device 4 for processing the measurements taken with the probes, a base 5 for holding the cups 2, 3 and for establishing a measuring circuit between the cups 2, 3 and the processing device 4, the processing device 4 being programmed for carrying the steps d) to i) of the inventive method. The equipment can comprise a third cup 6 for determining other parameters of the cast alloy.

In this text, the term "comprises" and its derivations (such as "comprising", etc.) should not be understood in an excluding sense, that is, these terms should not be interpreted as excluding the possibility that what is described and defined may include further elements, steps, etc.

The invention is obviously not limited to the specific embodiment(s) described herein, but also encompasses any variations that may be considered by any person skilled in the art (for example, as regards the choice of materials, dimensions, components, configuration, etc.), within the general scope of the invention as defined in the claims.

## Claims

1. Method to determine the carbon equivalent content of a cast iron alloy which comprises the following steps:

a) Previously determining (1.1) the maximum carbon equivalent value for the validity of a linear thermal analysis model for hypoeutectic and eutectic zones that relates the carbon equivalent with the liquidus temperature and the eutectic minimum temperature of a white iron solidification curve;

b) Previously determining (1.2) the minimum carbon equivalent value for the validity of a linear thermal analysis model for hypereutectic zone that relates the carbon equivalent with the liquidus temperature of a graphitic solidification curve and the eutectic minimum temperature of a white iron solidification curve;

c) Pour a liquid first sample (1.3) of the iron alloy in a composition cup and a liquid second sample (I.4) of the iron alloy in a thermal cup;

d) Obtain the solidification curve for both the first sample (1.5) and the second sample (1.6) and hence the white iron solidification curve and the graphitic solidification curve, respectively;

e) Obtain (1.7) from the solidification curve of the first sample the liquidus temperature and the eutectic minimum temperature, and with these two values, define the carbon equivalent content for the iron alloy by means of the linear thermal analysis model for hypoeutectic and eutectic zones;

f) If the carbon equivalent content for the iron alloy obtained in step e) is lower than the maximum carbon equivalent value defined in a), then establish (1.8) the carbon equivalent content of the iron alloy by using the linear model for hypoeutectic and eutectic zones;

g) If (1.8) the carbon equivalent content for the iron alloy obtained in step e) is higher than the maximum carbon equivalent value defined in a), then obtain (1.9) the liquidus temperature of the second sample and check (1.10) if it is higher than the value of the liquidus temperature corresponding to the minimum carbon equivalent value defined in b);

h) If the comparisons of step g) are positive, then establish the carbon equivalent content of the cast iron alloy by using the linear thermal analysis model for hypereutectic zone (1.11);

i) If the second comparison (1.10) of step g) is negative, then establish the carbon equivalent as the one obtained in e).

2. Method according to claim 1, wherein steps a) and b) are carried out by obtaining the solidification curves for multiple alloys having different carbon equivalent contents and determining the carbon and silicon contents by using techniques not making use of the solidification curves.

3. Method according to any of the previous claims, wherein step e) is carried out by determining the first inflexion point occurring before the eutectic plateau or by detecting a peak in the first derivative higher than a predetermined threshold value and before the peak described for the eutectic plateau, obtain the eutectic minimum temperature, by detecting the first null point or peak of the first derivative on the eutectic plateau.

4. Method according to any of the previous claims, wherein step g) is carried out by determining the first inflexion point occurring before the eutectic plateau or by detecting a peak in the first derivative higher than a predetermined threshold value and before the peak described for the eutectic plateau.

5. Method according to any of the previous claims, wherein the linear thermal analysis model for hypoeutectic and eutectic zones that relates the carbon content with the liquidus temperature (TL) and the eutectic minimum temperature (TE) for a white iron is:

$$\%C = 0.0178 \cdot TE - 0.0084 \cdot TL - 6.51 \qquad (III)$$

6. Method according to any of the previous claims, wherein the linear thermal analysis model for hypoeutectic and eutectic zones that relates the silicon content with the eutectic minimum temperature (TE) for a white iron is:

$$\%Si = 78.23 - 0.0683 \cdot TE \qquad (II)$$

7. Method according to any of the previous claims, wherein the linear thermal analysis model for hypereutectic zone that relates the carbon content with the liquidus temperature of a graphitic solidification curve (TLS) and the eutectic minimum temperature (TE) for a gray iron solidification curve:

$$\%C = 0.0082 \cdot TE - 0.0019 \cdot TLS - 7.382 \qquad (IV)$$

8. Method according to any of the previous claims, wherein the minimum value of the liquidus temperature of a graphitic solidification curve of validity of a linear thermal analysis model for hypereutectic zone that relates the carbon equivalent with the liquidus temperature for a graphitic solidification curve and the eutectic minimum temperature of a gray solidification curve is 1164ºC.

9.  Method to determine the carbon equivalent content of a cast iron alloy having a hypereutectic composition which comprises the following steps:

   a) Pour a liquid first sample of the iron alloy in a composition cup and a liquid second sample in a thermal cup;
   b) Obtain the solidification curve for both the first sample and the second sample;
   c) If the liquidus temperature can be determined for both samples and they are not close to the eutectic minimum temperature then apply a linear thermal analysis model for hypoeutectic and eutectic zones that relates the carbon equivalent with the liquidus temperature and the eutectic minimum temperature of a white iron solidification curve and obtain the corresponding carbon equivalent content;
   d) If the liquidus temperature can only be determined for the first sample and is close to the eutectic minimum temperature and the second sample does not describe the liquidus temperature, then apply a linear thermal analysis model for hypoeutectic and eutectic zones that relates the carbon equivalent with the liquidus temperature and the eutectic minimum temperature of a white iron solidification curve and obtain the corresponding equivalent carbon content;
   e) If the liquidus temperature cannot be determined for neither of the first and second samples then apply a linear thermal analysis model for hypoeutectic and eutectic zones.
   f) If the liquidus temperature can only be determined for the second sample and the liquidus temperature is above a determined threshold value, then apply a linear thermal analysis model for hypereutectic zone.

10. Method according to claim 9, wherein the linear thermal analysis model for hypoeutectic and eutectic zones that relates the carbon content with the liquidus temperature (TL) and the eutectic minimum temperature (TE) for a white iron is:

$$\%C = -6.51 - 0.0084*TL + 0.0178*TE \qquad (III)$$

11. Method according to claims 9 or 10, wherein the linear thermal analysis model for hypoeutectic and eutectic zones that relates the silicon content with the eutectic minimum temperature (TE) for a white iron is:

$$\%Si = 78.23 - 0.0683 \cdot TE \qquad (II)$$

12. Method according to any of claims 9 to 11, wherein the linear thermal analysis model for hypereutectic zone that relates the carbon content with the liquidus temperature of a graphitic solidification curve (TLS) and the eutectic minimum temperature (TE) for a white iron solidification curve is:

$$\%C = 0.0082 \cdot TE - 0.0019 \cdot TLS - 7.382 \qquad (IV)$$

13. Method according to any of claims 9 to 12, wherein the minimum value of the liquidus temperature of a graphitic solidification curve of validity of a linear thermal analysis model for hypereutectic zone that relates the carbon equivalent with the liquidus temperature for a graphitic solidification curve and the eutectic minimum temperature of a gray solidification curve is 1164ºC.

14. Equipment for determining the carbon equivalent content of a cast iron alloy having a hypereutectic composition, which comprises a composition cup (2), a thermal cup (3), both provided with temperature probes, a processing device (4) for processing the measurements taken with the probes, a base (5) for holding the cups (2, 3) and for establishing a measuring circuit between the cups (2, 3) and the processing device (4), the processing device (4) being programmed for carrying out the steps d) to i) of the method of claims 1 to 8 or steps d) to f) of the method of claims 9 to 13, respectively.

**Patentansprüche**

1. Verfahren zur Bestimmung des Kohlenstoffäquivalentgehalts einer Gusseisenlegierung, mit den folgenden Schritten:

   a) Vorherige Bestimmung (1.1) eines maximalen Kohlenstoffäquivalenzwertes für die Gültigkeit eines linearen Thermoanalysemodells für untereutektische und eutektische Zonen, das das Kohlenstoffäquivalent mit einer Liquidustemperatur und einer eutektischen Mindesttemperatur einer Gusseisenerstarrkurve in Beziehung setzt;
   b) vorherige Bestimmung (1.2) des minimalen Kohlenstoffäquivalenzwertes für die Gültigkeit eines linearen Thermoanalysemodells für übereutektische Zone, das das Kohlenstoffäquivalent mit der Liquidustemperatur einer graphitischen Erstarrungskurve und der eutektischen Mindesttemperatur der Gusseisenerstarrkurve in Beziehung setzt;
   c) Gießen einer flüssigen ersten Probe (1.3) der Eisenlegierung in einen Mischbecher und einer flüssigen zweiten Probe (1.4) der Eisenlegierung in einen Wärmetiegel;
   d) Ermitteln der Erstarrungskurve sowohl für die erste Probe (1.5) als auch für die zweite Probe (1.6) und somit der Erstarrungskurve des Gusseisens bzw. der graphitischen Erstarrungskurve;
   e) Ermitteln (1.7) der Liquidustemperatur und der eutektischen Mindesttemperatur aus der Erstarrungskurve der ersten Probe und mit diesen beiden Werten den Kohlenstoffäquivalentgehalt für die Eisenlegierung mit Hilfe des linearen Thermoanalysemodells für untereutektische und eutektische Zone definieren;
   f) Wenn der in Schritt e) erhaltene Kohlenstoffäquivalentgehalt für die Eisenlegierung niedriger ist als der in a) definierte maximale Kohlenstoffäquivalentwert, wird der Kohlenstoffäquivalentgehalt der Eisenlegierung mit Hilfe des linearen Modells für untereutektische und eutektische Zonen bestimmt (1.8);
   g) Wenn (1.8) der Kohlenstoffäquivalentgehalt für die in Schritt e) erhaltene Eisenlegierung höher ist als der unter a) definierte maximale Kohlenstoffäquivalenzwert, wird die Liquidustemperatur der zweiten Probe ermittelt (1.9) und geprüft (1.10), ob diese höher ist als der Wert der Liquidustemperatur, der dem unter b) definierten minimalen Kohlenstoffäquivalenzwert entspricht;
   h) wenn die Vergleiche von Schritt g) positiv sind, wird der Kohlenstoffäquivalenzwert der Gusseisenlegierung unter Verwendung des linearen Thermoanalysemodells für die übereutektische Zone bestimmt (1.11);
   i) Wenn der zweite Vergleich (1.10) von Schritt g) negativ ist, wird der Kohlenstoffäquivalenzwert wie in Schritt e) bestimmt.

2. Verfahren nach Anspruch 1, wobei die Schritte a) und b) durchgeführt werden, unter Einbeziehung von Erstarrungskurven für mehrere Legierungen mit unterschiedlichen Kohlenstoffäquivalentgehalten und Bestimmung der Kohlenstoff- und Siliziumgehalte unter Verwendung von Techniken, die die Erstarrungskurven nicht verwenden.

3. Verfahren nach einem der vorhergehenden Ansprüche, wobei Schritt e) durch Bestimmung eines ersten Wendepunktes, der vor einem eutektischen Plateau auftritt, oder durch Detektion eines Peaks in der ersten Ableitung, der höher als ein vorbestimmter Schwellenwert ist und vor dem das eutektische Plateau beschreibenden Peak liegt, durch Erfassen der eutektischen Mindesttemperatur, indem der erste Nullpunkt oder das Peak der ersten Ableitung auf dem eutektischen Plateau detektiert wird, durchgeführt wird.

4. Verfahren nach einem der vorhergehenden Ansprüche, wobei Schritt g) durch Bestimmung des ersten Wendepunktes, der vor dem eutektischen Plateau auftritt, oder durch Detektion eines Peaks in der ersten Ableitung, der höher als ein vorbestimmter Schwellenwert und vor dem für das eutektische Plateau beschriebenen Peak liegt, durchgeführt wird.

5. Verfahren nach einem der vorhergehenden Ansprüche, wobei das lineare Thermoanalysemodell für untereutektische und eutektische Zonen, das den Kohlenstoffgehalt mit der Liquidustemperatur (TL) und der eutektischen Mindesttemperatur (TE) für ein Gusseisen in Beziehung setzt, lautet:

$$\%C = 0.0178 \cdot TE - 0.0084 \cdot TL - 6.51 \ (III)$$

6. Verfahren nach einem der vorhergehenden Ansprüche, wobei das lineare Thermoanalysemodell für untereutektische und eutektische Zonen, das den Siliziumgehalt mit der eutektischen Mindesttemperatur (TE) für ein Gusseisen in Beziehung setzt, lautet:

$$\%Si = 78.23 - 0.0683 \cdot TE \ (II)$$

7. Verfahren nach einem der vorhergehenden Ansprüche, bei dem das lineare Thermoanalysemodell für übereutektische Zonen, das den Kohlenstoffgehalt mit der Liquidustemperatur einer graphitischen Erstarrungskurve (TLS) und der eutektischen Mindesttemperatur (TE) für eine Grauguss-Erstarrungskurve in Beziehung setzt, lautet:

$$\%C = 0.0082 \cdot TE - 0.0019 \cdot TLS - 7.382 \ (IV)$$

8. Verfahren nach einem der vorhergehenden Ansprüche, wobei der Mindestwert der Liquidustemperatur einer graphitischen Erstarrungskurve einer Geltung eines linearen thermischen Analysemodells für übereutektische Zonen, das das Kohlenstoffäquivalent mit der Liquidustemperatur für eine graphitische Erstarrungskurve und die eutektische Mindesttemperatur einer Grauguss-Erstarrungskurve in Beziehung setzt, 1164°C beträgt.

9. Verfahren zur Bestimmung des Kohlenstoffäquivalentgehalts einer Gusseisenlegierung mit einer übereutektischen Zusammensetzung, das die folgenden Schritte umfasst:

a) Gießen einer flüssigen ersten Probe der Eisenlegierung in einen Mischbecher und einer flüssigen zweiten Probe in einen Wärmetiegel;
b) Ermitteln der Erstarrungskurve sowohl für die erste Probe als auch für die zweite Probe;
c) Wenn die Liquidustemperatur für beide Proben bestimmt werden kann und diese nicht in der Nähe der eutektischen Mindesttemperatur liegen, wird ein lineares thermisches Analysemodell für untereutektische und eutektische Zonen angewendet, das das Kohlenstoffäquivalent mit der Liquidustemperatur und der eutektischen Mindesttemperatur einer Erstarrungskurve für Gusseisen in Beziehung setzt, um den entsprechenden Kohlenstoffäquivalentgehalt zu ermitteln;
d) Wenn die Liquidustemperatur nur für die erste Probe bestimmt werden kann und nahe der eutektischen Mindesttemperatur liegt und die zweite Probe nicht die Liquidustemperatur ausgibt, wird ein lineares Thermoanalysemodell für untereutektische und eutektische Zonen angewendet, das das Kohlenstoffäquivalent mit der Liquidustemperatur und der eutektischen Mindesttemperatur einer GusseisenErstarrkurve in Beziehung setzt, um den entsprechenden Kohlenstoffäqivalenzgehalt zu ermitteln;
e) Wenn die Liquidustemperatur weder für die erste noch für die zweite Probe bestimmt werden kann, wird ein lineares Thermoanalysemodell für untereutektische und eutektische Zonen angewendet.
f) Wenn die Liquidustemperatur nur für die zweite Probe bestimmt werden kann und die Liquidustemperatur über einem bestimmten Schwellenwert liegt, wird ein lineares Thermoanalysemodell für die übereutektische Zone angewendet.

10. Verfahren nach Anspruch 9, wobei das lineare Thermoanalysemodell für untereutektische und eutektische Zonen, das den Kohlenstoffgehalt mit der Liquidustemperatur (TL) und der eutektischen Mindesttemperatur (TE) für ein Gusseisen in Beziehung setzt, lautet:

$$\%C = -6.51 - 0.0084*TL + 0.0178*TE \ (III)$$

11. Verfahren nach Anspruch 9 oder 10, wobei das lineare Thermoanalysemodell für untereutektische und eutektische Zonen, das den Siliziumgehalt mit der eutektischen Mindesttemperatur (TE) für ein Gusseisen in Beziehung setzt, lautet:

$$\%Si = 78.23 - 0.0683 \cdot TE \ (II)$$

12. Verfahren nach einem der Ansprüche 9 bis 11, bei dem das lineare Thermoanalysemodell für die übereutektische Zone, das den Kohlenstoffgehalt mit der Liquidustemperatur einer graphitischen Erstarrungskurve (TLS) und der eutektischen Mindesttemperatur (TE) für eine Gusseisen-Erstarrungskurve in Beziehung setzt, lautet:

$$\%C = 0.0082 \cdot TE - 0.0019 \cdot TLS - 7.382 \ (IV)$$

13. Verfahren nach einem der Ansprüche 9 bis 12, wobei der Mindestwert der Liquidustemperatur einer graphitischen Erstarrungskurve einer Geltung eines linearen Thermoanalysemodells für übereutektische Zone, das das Kohlenstoffäquivalent mit der Liquidustemperatur für eine graphitische Erstarrungskurve und die eutektische Mindesttemperatur einer Grauguss-Erstarrungskurve in Beziehung setzt, 1164°C beträgt.

14. Vorrichtung zur Bestimmung des Kohlenstoffäquivalentgehalts einer Gusseisenlegierung mit einer übereutektischen Zusammensetzung, die einen Mischbecher (2), einen Wärmetiegel (3), beide mit Temperatursonden versehen, und eine Verarbeitungsvorrichtung (4) zur Verarbeitung der mit Sonden durchgeführten Messungen umfasst, eine Basis (5) zum Halten der Becher (2, 3) und zum Herstellen eines Messkreises zwischen den Bechern (2, 3) und der Verarbeitungsvorrichtung (4), wobei die Verarbeitungsvorrichtung (4) so programmiert ist, dass sie die Schritte d) bis i) des Verfahrens nach den Ansprüchen 1 bis 8 bzw. die Schritte d) bis f) des Verfahrens nach den Ansprüchen 9 bis 13 ausführt.

## Revendications

1. Méthode pour déterminer la teneur en équivalent carbone d'un alliage de fonte, qui comprend les étapes suivantes :

    a) au préalable la détermination (1.1) de la valeur d'équivalent carbone maximale pour la validité d'un modèle d'analyse thermique linéaire pour des zones hypo-eutectiques et eutectiques qui concerne l'équivalent carbone avec la température de liquidus et la température minimale eutectique d'une courbe de solidification de fer blanc ;
    b) au préalable la détermination (I.2) de la valeur d'équivalent carbone minimale pour la validité d'un modèle d'analyse thermique linéaire pour une zone hyper-eutectique qui concerne l'équivalent carbone avec la température de liquidus d'une courbe de solidification graphitique et la température minimale eutectique d'une courbe de solidification de fer blanc ;
    c) le versement d'un premier échantillon liquide (I.3) de l'alliage de fer dans une coupelle de composition et d'un deuxième échantillon liquide (I.4) de l'alliage de fer dans une coupelle thermique ;
    d) l'obtention de la courbe de solidification pour tant le premier échantillon (I.5) que le deuxième échantillon (I.6) et ainsi la courbe de solidification de fer blanc et la courbe de solidification graphitique, respectivement ;
    e) l'obtention (I.7), à partir de la courbe de solidification du premier échantillon, de la température de liquidus et de la température minimale eutectique, et, avec ces deux valeurs, la définition de la teneur en équivalent carbone pour l'alliage de fer au moyen du modèle d'analyse thermique linéaire pour des zones hypo-eutectiques et eutectiques ;
    f) si la teneur en équivalent carbone pour l'alliage de fer obtenu dans l'étape e) est inférieure à la valeur en équivalent carbone maximale définie en a), alors l'établissement (I.8) de la teneur en équivalent carbone de l'alliage de fer par utilisation du modèle linéaire pour des zones hypo-eutectiques et eutectiques ;
    g) si (I.8) la teneur en équivalent carbone pour l'alliage de fer obtenu dans l'étape e) est supérieure à la valeur en équivalent carbone maximale définie en a), alors l'obtention (I.9) de la température de liquidus du deuxième échantillon et la vérification (1.10) si elle est supérieure à la valeur de la température de liquidus correspondant à la valeur en équivalent carbone minimale définie en b) ;
    h) si les comparaisons de l'étape g) sont positives, alors l'établissement de la teneur en équivalent carbone de l'alliage de fonte par utilisation du modèle d'analyse thermique linéaire pour une zone hyper-eutectique (1.11) ;
    i) si la deuxième comparaison (I.10) de l'étape g) est négative, alors l'établissement de l'équivalent carbone comme étant celui obtenu en e).

2. Méthode selon la revendication 1, dans laquelle les étapes a) et b) sont mises en œuvre par l'obtention des courbes de solidification pour de multiples alliages ayant différentes teneurs en équivalent carbone et la détermination des teneurs en carbone et en silicium par l'utilisation de techniques n'utilisant pas les courbes de solidification.

3. Méthode selon l'une quelconque des revendications précédentes, dans laquelle l'étape a) est mise en œuvre par la détermination du premier point d'inflexion apparaissant avant le plateau eutectique ou par la détection d'un pic dans la première dérivée supérieur à une valeur de seuil prédéterminée et avant le pic décrit pour le plateau eutectique, l'obtention de la température minimale eutectique, par la détection du premier pic ou point nul de la première dérivée sur le plateau eutectique.

4. Méthode selon l'une quelconque des revendications précédentes, dans laquelle l'étape g) est mise en œuvre par la détermination du premier point d'inflexion apparaissant avant le plateau eutectique ou par la détection d'un pic dans la première dérivée supérieur à une valeur de seuil prédéterminée et avant le pic décrit pour le plateau eutectique.

5. Méthode selon l'une quelconque des revendications précédentes, dans laquelle le modèle d'analyse thermique linéaire pour des zones hypo-eutectiques et eutectiques qui concerne la teneur en carbone avec la température de liquidus (TL) et la température minimale eutectique (TE) pour un fer blanc est :

$$\% \, C = 0{,}0178 \cdot TE - 0{,}0084 \cdot TL - 6{,}51 \qquad (III)$$

6. Méthode selon l'une quelconque des revendications précédentes, dans laquelle le modèle d'analyse thermique linéaire pour des zones hypo-eutectiques et eutectiques qui concerne la teneur en silicium avec la température minimale eutectique (TE) pour un fer blanc est :

$$\% \, Si = 78{,}23 - 0{,}0683 \cdot TE \qquad (II)$$

7. Méthode selon l'une quelconque des revendications précédentes, dans laquelle le modèle d'analyse thermique linéaire pour une zone hyper-eutectique qui concerne la teneur en carbone avec la température de liquidus d'une courbe de solidification graphitique (TLS) et la température minimale eutectique (TE) pour une courbe de solidification de fonte grise est :

$$\% \, C = 0{,}0082 \cdot TE - 0{,}0019 \cdot TLS - 7{,}382 \qquad (IV)$$

8. Méthode selon l'une quelconque des revendications précédentes, dans laquelle la valeur minimale de la température de liquidus d'une courbe de solidification graphitique de validité d'un modèle d'analyse thermique linéaire pour une zone hyper-eutectique qui concerne l'équivalent carbone avec la température de liquidus pour une courbe de solidification graphitique et la température minimale eutectique d'une courbe de solidification de fonte grise est de 1164 °C.

9. Méthode pour déterminer la teneur en équivalent carbone d'un alliage de fonte ayant une composition hyper-eutectique, qui comprend les étapes suivantes :

   a) le versement d'un premier échantillon liquide de l'alliage de fer dans une coupelle de composition et d'un deuxième échantillon liquide dans une coupelle thermique ;
   b) l'obtention de la courbe de solidification pour le premier échantillon et le deuxième échantillon ;
   c) si la température de liquidus peut être déterminée pour les deux échantillons et que ces températures ne sont pas proches de la température minimale eutectique, alors l'application d'un modèle d'analyse thermique linéaire pour des zones hypo-eutectiques et eutectiques qui concerne l'équivalent carbone avec la température de liquidus et la température minimale eutectique d'une courbe de solidification de fer blanc et l'obtention de la teneur en équivalent carbone correspondante ;
   d) si la température de liquidus peut être déterminée seulement pour le premier échantillon et est proche de la température minimale eutectique, et le deuxième échantillon ne décrit pas la température de liquidus, alors l'application d'un modèle d'analyse thermique linéaire pour des zones hypo-eutectiques et eutectiques qui concerne l'équivalent carbone avec la température de liquidus et la température minimale eutectique d'une courbe de solidification de fer blanc et l'obtention de la teneur en équivalent carbone correspondante ;
   e) si la température de liquidus ne peut être déterminée ni pour le premier ni pour le deuxième des échantillons, alors l'application d'un modèle d'analyse thermique linéaire pour des zones hypo-eutectiques et eutectique ;
   f) si la température de liquidus peut être déterminée seulement pour le deuxième échantillon et la température de liquidus dépasse une valeur de seuil déterminée, alors l'application d'un modèle d'analyse thermique linéaire pour une zone hyper-eutectique.

10. Méthode selon la revendication 9, dans laquelle le modèle d'analyse thermique linéaire pour des zones hypo-eutectiques et eutectiques qui concerne l'équivalent carbone avec la température de liquidus (TL) et la température minimale eutectique (TE) pour un fer blanc est :

$$\% \, C = -6{,}51 - 0{,}0084*TL + 0{,}0178*TE \qquad (III)$$

11. Méthode selon la revendication 9 ou 10, dans laquelle le modèle d'analyse thermique linéaire pour des zones hypo-eutectiques et eutectiques qui concerne la teneur en silicium avec la température minimale eutectique (TE) pour un fer blanc est :

$$\% \, Si = 78,23 - 0,0683 \cdot TE \qquad\qquad (II)$$

12. Méthode selon l'une quelconque des revendications 9 à 11, dans laquelle le modèle d'analyse thermique linéaire pour une zone hyper-eutectique qui concerne la teneur en carbone avec la température de liquidus d'une courbe de solidification graphitique (TLS) et la température minimale eutectique (TE) pour une courbe de solidification de fer blanc est :

$$\% \, C = 0,0082 \cdot TE - 0,0019 \cdot TLS - 7,382 \qquad\qquad (IV)$$

13. Méthode selon l'une quelconque des revendications 9 à 12, dans laquelle la valeur minimale de la température de liquidus d'une courbe de solidification graphitique de validité d'un modèle d'analyse thermique linéaire pour une zone hyper-eutectique qui concerne l'équivalent carbone avec la température de liquidus pour une courbe de solidification graphitique et la température minimale eutectique d'une courbe de solidification de fonte grise est de 1164 °C.

14. Equipement pour déterminer la teneur en équivalent carbone d'un alliage de fonte ayant une composition hyper-eutectique, qui comprend une coupelle de composition (2), une coupelle thermique (3), les deux dotées de sondes de température, un dispositif de traitement (4) pour traiter les mesures prises avec les sondes, une base (5) pour maintenir les coupelles (2, 3) et pour établir un circuit de mesure entre les coupelles (2, 3) et le dispositif de traitement (4), le dispositif de traitement (4) étant programmé pour réaliser les étapes d) à i) de la méthode des revendications 1 à 8 ou les étapes d) à f) de la méthode des revendications 9 à 13, respectivement.

FIG. 1

**FIG. 2**

FIG. 3

FIG. 4

**FIG. 5**

FIG. 6

FIG. 7

**FIG. 8**

FIG. 9

FIG. 10

**FIG. 11**

FIG. 12

**FIG. 13**

**FIG. 14**

**FIG. 15**

**FIG. 16**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3546921 A
- US 4008604 A
- US 5577545 A
- US 5503475 A

**Non-patent literature cited in the description**

- Thermal Analysis of Cast Iron. Heraeus Electro-Nite
- COMPUTER-AIDED DIFFERENTIAL THERMAL ANALYSIS OF SPHEROIDAL AND COMPACTED GRAPHITE CAST IRONS. **CHEN IG et al.** AFS TRANSACTIONS. AMERICAN FOUNDRY SOCIE-TY, April 1984, vol. 92, 947-964